# EUROPEAN PATENT APPLICATION

(11) **EP 1 645 254 A1**
(43) Date of publication of application: **12.04.2006**
(21) Application number: 04445107.8
(22) Date of filing: 11.10.2004
(51) Int. Cl.: A61F 13/08, A61H 23/02, A61B 17/135

(54) **Electro active compression bandage**

(71) Applicant: SMM Medical AB, 582 16 Linköping (SE)
(72) Inventor: Larson, Tomas, 590 55 Sturefors (SE); Toth, Landy, 582 18 Linköping (SE)
(74) Representative: Wihlsson, Joakim Per Magnus

(57) **Abstract**

The invention relates to exertion of an external pressure to a human body part (100). A proposed device includes a number of segments (S1, S2) adapted to at least partially enclose the body part (100) in a form-fitting manner. Each segment (S1, S2) contains a controllable active-material based actuator, which is adapted to cause the segment (S1, S2) to apply a basic pressure profile (P1; P2) to the body part (100) in response to a control signal. A pressure transition system (PTS) is adapted to redistribute the basic pressure profile (P1) of at least one of the segments (S1, S2), such that in response to a control signal in respect of this segment (S1) an adjusted pressure profile (P1_{adj}) different from the basic pressure profile (P1) is applied to the body part.

## Description

### THE BACKGROUND OF THE INVENTION AND PRIOR ART

The present invention relates generally to the application of pressure profiles to living tissues. More particularly the invention relates to a device for exerting an external pressure to a human body part according to the preamble of claim 1 and a therapeutic garment according to the preamble of claim 28.

External pressure profiles may be applied to living tissues, e.g. of a person's limb, in order to attain various effects with respect to these tissues. Perhaps the most well known example is the so-called G-suit worn by a pilot to restrict the blood circulation in his/her lower body parts under certain conditions, and thus reduce the risk that an insufficient amount of blood is fed to the pilot's head.

However, also in the medical field there are many examples of situations in which it is relevant/desirable to apply an external pressure to a part of the human body, in order to cure or mitigate a disease or condition. For instance, lymphoedema is a condition where the lymphatic system of a patient has been compromised, thereby resulting in a buildup of lymphatic fluids and proteins in one or more extremities. So far, various approaches have been attempted to control the swelling of the afflicted extremities. The compression-based treatment of lymphoedema is primarily dealt with in three ways, which may be combined to achieve an improved result, compression bandaging, pneumatic compression pumps and massage. The compression bandaging may be further divided into two general approaches: multi-layered lymphatic bandaging and elastic compression garments/ bandages. Both these methods are used to statically compress the afflicted limbs, whereas pneumatic compression pumps and massage represent dynamic treatments.

Multi-layered lymphatic bandages are applied to a patient in order to reshape one or more of the patient's limbs. The bandages consist of absorbent layers, padding and short-stretch bandages. The absorbent layers must be custom made by a technician to fit the patient. Moreover, the underlying pressure is unknown after application, and the bandages are there to prevent the limb from further expanding and to breakup proteins with the help of patient movement. Nevertheless, these bandages are associated with numerous problems. During the treatment the bandages must be adjusted many times, for example because the bandages have a static shape and the shape of the limb varies over time. The bandages are also bulky and hot to wear due to the many layers applied to the limb, and therefore the bandages cannot be worn under clothing. Naturally, the therapy for the patient is limited in that the multi-layered lymphatic bandages cannot actively pressurize the body.

Elastic compression bandages are used to statically pressurize an afflicted limb. Here, a caregiver wraps the afflicted limb with a combination of elastic bandages and absorbent layers. The bandages are arranged so as to apply a graduated pressure to the limb. The pressure gradient along the limb is structured such that the highest pressure is at the distal end, and the lowest pressure is located at the proximal end of the limb. Hence, also in this case, the pressure application is static and a qualified person must apply the bandages to ensure that an appropriate pressure is accomplished, particularly since there is no convenient way to accurately measure the pressure applied to the limb. Normally, a constant bandage tension is applied while wrapping the limb, and the pressure graduation is typically a consequence of the limb being thinner at the distal part than at the proximal part. As the limb changes size due to the pressure, and as the bandages creep, the pressure application will decrease. This is true already within hours of applying the bandages.

A pneumatic compression pump device is used to dynamically pressurize limbs of patients. Here, dynamic pressurization is employed both to pump lymphatic fluids from the limb in wavelike, or graduated, pressure profiles and to breakup proteins that collect and harden in the afflicted limb. To generate the wavelike and graduated pressure profiles along the limb, a sleeve portion of the device must have multiple chambers. Each chamber is pressurized at the appropriate time as determined by the treatment prescription. However, the pneumatic compression pump devices are relatively inefficient, and therefore cannot operate from batteries for any significant length of time. In fact, it is normally required that the device be connected to mains power, and as a further consequence that the patient be stationary during the treatment. Since the pneumatic compression pump device is airtight, heat produced by the patient is accumulated in the device. Thus, the device can only be used for comparatively short durations before it becomes too uncomfortable for the patient. Although the device can dramatically reduce oedema during treatment, after use, static compression bandages (or equivalent) must be applied to prevent the fluids from draining back into the afflicted limb. Additionally, the device is noisy, the air-pressure measurements used to infer pressure applied to the limb can be inaccurate, and unintentionally high pressure levels may harm the patient.

A qualified massage therapist/clinician may also apply various forms of massage to a patient. Such massage techniques are highly technical and require significant training to perform. Thus, the outcome of the treatment depends very much on the skill of the clinician.

U.S. patent No. 5,997,465 describes a device for exerting an external pressure on a human body, wherein the device surrounds a body part with a comfortable fit. The device includes memory material components, which alter their shape in response to an electric signal. Thereby, in a contracted state, these components may squeeze the body part, for example to prevent pooling of blood in the body part of a pilot when subjected to G-forces. Then, in a non-contracted state (i.e. when no electric signal is present) the memory material components resume their original shape, and the squeezing ceases.

The electrical control proposed in this document overcomes some of the shortcomings associated with the above-described dynamic procedures, i.e. the pneumatic compression pump devices and massage forms. However, the solution is still inadequate for many medical applications. For instance, the skin of a patient is often compromised due to various medical conditions. In addition, the health of the patient's skin may lack elasticity, strength and resilience. Therefore, extreme care must be given to ensure that the pressure profile applied to the patient is medically safe. For instance, if highly localized pressures are applied for long periods of time, the tissues can tear and/or pressure ulcers may be formed. Moreover, if subjected to repeated rubbing, the skin can chafe, or even rip. Additionally, the medical treatments often require that the garments be worn for prolonged periods of time during which pressure and/or repeated pressure pulsation may be applied. Such activities further increase the risk of damage being caused to the patient's skin. Some medical applications may also require that the pressure profiles be variable over a very wide range, for example to promote fluid flow in superficial and interstitial tissues. Sometimes it is desired that the pressure profile emulate the naturally occurring function of a healthy body part.

### SUMMARY OF THE INVENTION

The object of the present invention is therefore to alleviate the above-mentioned problems and thus accomplish improved pressure profiles in terms of a well-defined location, distribution and magnitude of the pressure that is applied to a human body part.

According to one aspect of the invention, the object is achieved by the initially described device, wherein the device further includes a pressure transition system adapted to redistribute the basic pressure profile of at least one of said segments. By means of this system, an adjusted pressure profile different from the basic pressure profile will be applied to the body part in response to a control signal in respect of the at least one segment.

An important advantage attained thereby is that very flexible pressure profiles may be accomplished, which are adapted to suit the needs of various treatments. The proposed pressure transition system also facilitates pressure application in regions of the body not amendable to direct application from actuators, e.g. elbows and wrists. Thus, as a further consequence, patient mobility is facilitated during treatment. Also individual patient needs may be handled, such as in cases where the skin is extremely vulnerable.

According to one preferred embodiment of this aspect of the invention, the device includes at least two segments. A first segment is adapted to at least partially enclose a first portion of the body part and a second segment is adapted to at least partially enclose a second portion of the body part. Moreover, the pressure transition system is adapted to redistribute pressure profiles between the first and second segments, such that a control signal in respect of the first segment causes the pressure transition system to apply an adjusted pressure profile to at least a part of the second portion of the body part. Correspondingly, a control signal in respect of the second segment causes the pressure transition system to apply an adjusted pressure profile to at least a part of the first portion of the body part. Thereby, the pressure profiles associated with the segments are smoothed out, so that the patient senses relatively fuzzy pressures. This generally enhances the patient comfort during the treatment. More important, however, the efficacy of the treatment is improved across joints and other complicated body regions where actuators cannot directly apply pressure.

According to another preferred embodiment of this aspect of the invention, the pressure transition system is adapted to be positioned between the first and second segments when the device is fitted on the body part. This location of the pressure transition system is advantageous, since it enables pressure bridging between the segments. At the same time, the device may have a comparatively thin cross section.

According to yet another preferred embodiment of this aspect of the invention, the pressure transition system is adapted to be positioned between a first surface defined by the first and second segments and a second surface defined by the body part. The pressure transition system here extends over the first and second portions of the body part when the device is fitted on the body part. Consequently, a pressure profile generated by the first segment may efficiently "leak over" to the second portion of the body part via the pressure transition system, and vice versa.

Preferably the pressure transition system also has a low-friction surface towards the first and second segments. The surface is thereby adapted to allow a smooth tangential movement of the first and second segments relative to the pressure transition system. This design is advantageous because it mitigates any undesired effects on the body part, such as friction, caused by relative movements created by the segments' actuators.

According to still another preferred embodiment of this aspect of the invention, the device includes at least two segments of which a first segment is adapted to at least partially enclose a first portion of the body part, and a second segment is adapted to at least partially enclose a- second portion of the body part.

Here, the first and second segments are arranged such that a portion of the first segment covers a portion of the second segment when the device is fitted on the body part. Thereby, an alternative, or complementary, means is provided for accomplishing a pressure profile leak-over between different segments and to attain smoothed-out/fuzzy pressure profiles.

According to another preferred embodiment of this aspect of the invention, the pressure transition system includes a number of collapsible ribs, which are adapted to extend along a general central axis of the body part. The ribs are positioned between at least one segment and a particular portion of the body part when the device is fitted on the body part. An actuator in each of the at least one segment is adapted to cause a tangential movement of the segment relative to the body part and the collapsible ribs are adapted to fold in response to this movement, such that when folded the ribs exert a radial pressure on the particular portion of the body part. Such a transformation between a tangential movement and a radial pressure is desirable because it allows a design with a very slim device profile.

Alternatively, or as a complement thereto, the pressure transition system may include at least one flexible chamber, which is adapted to be positioned between at least one of the segments and a particular portion of the body part when the device is fitted on the body part. An actuator in each of the at least one segment is adapted to cause a tangential movement of segment relative to the body part, and the at least one chamber is adapted to transform this movement into a resulting radial pressure on the particular portion of the body part.

According to yet another preferred embodiment of this aspect of the invention, the flexible chamber has an elastic wall of an anisotropic material, and the chamber is arranged relative to the body part when the device is fitted on the body part, such that the chamber is relatively stretchable in a circumferential direction of the body part and relatively stiff in a direction along a general central axis of the body part. Thus, any tension forces generated by the actuators may efficiently be transformed into desired pressure profiles with respect to the body part.

According to still another preferred embodiment of this aspect of the invention, the pressure transition system includes a number of protrusions adapted to be positioned between at least one of the segments and a particular portion of the body part when the device is fitted on the body part. The protrusions, in turn, are adapted to convert the basic pressure profile of the at least one segment into a non-uniform pressure profile to the particular portion of the body part. For example, the protrusions may be cylindrical bulges. However, the protrusions may also include at least one rigid rib, which is adapted to extend along a general central axis of the body part when the device is fitted on the body part. Then, as the segment exerts a pressure profile, a respective peak pressure ridge is defined by a positioning of each of the at least one rib relative to the body part. Consequently, an increased pressure can be attained at one or more desired areas.

According to another preferred embodiment of this aspect of the invention, the above-mentioned control signal is an electrical signal. Moreover, each actuator has a morphology which is electrically adjustable, and a change in this morphology is instigated by the control signal. Hence, an actuator may originally have a particular morphology, or shape. Then, the actuator's morphology changes in response to an electrical control signal. Importantly, however, this morphology remains also *after* that the control signal ceased. Consequently, no electrical energy is required to maintain the thus altered actuator morphology. Of course, this is a highly desirable feature, since thereby for instance a static or quasi-static pressure may be applied very efficiently.

According to yet another preferred embodiment of this aspect of the invention, the device includes a control unit adapted to produce a respective control signal to each of segment. The control unit is adapted to vary the control signal over time, so that a particular treatment profile is implemented with respect to the body part. Preferably, the treatment profile involves producing repeated cycles of variations between relatively high and relatively low basic pressure profiles by means of each segment. Hence, the device may perform an intermittent compression therapy and/or adjust (e.g. increase) the applied pressure gradually.

According to still another preferred embodiment of this aspect of the invention, the pressure transition system includes a number of moisture passages adapted to receive exudates from the body part. Furthermore, the moisture passages (e.g. in the cells of an open-celled foam) may be adapted to transport (or milk) any received exudates from the body part to one or more liquid receptacles concomitantly with the repeating pressure cycles. Thereby, sweat can be removed from the patient's skin and exudates can be drawn from wounds.

According to another preferred embodiment of this aspect of the invention, the pressure transition system includes a number of air channels which are adapted to allow air to pass to the body part (e.g. via the cells of an open-celled foam). Preferably, also the air channels are adapted to exchange air between the body part and a local environment outside the device concomitantly with the repeating pressure cycles to improve the ventilation of the patient's skin.

According to yet another preferred embodiment of this aspect of the invention, the pressure transition system includes at least one sensor element adapted to register a physiological parameter of the body part. A data signal reflecting this parameter is transmitted to the control unit. Thereby, the control unit may survey and analyze the medical condition of the body part, and if necessary, trigger an alarm and/or alter the treatment profile.

According to still another preferred embodiment of this aspect of the invention, the pressure transition system includes at least one sensor element adapted to register a parameter expressing an environmental condition in proximity to the body part. A data signal reflecting this parameter is transmitted to the control unit. Thereby, the control unit may survey and analyze the environmental conditions for the body part, and if necessary, alter the treatment profile and/or trigger an alarm.

According to another preferred embodiment of this aspect of the invention, the pressure transition system includes at least one pocket adapted to contain a drug substance. The pressure transition system is also adapted to administer a transport of this substance to the body part. Thus, a pressure/massage treatment may be combined with a drug therapy. The drug substance may also be an antibacterial agent, so that the risk of infections and other sanitation related conditions could be reduced.

According to yet another preferred embodiment of this aspect of the invention, the drug substance is a gel adapted to perform a thermotherapy on the body part (i.e. either cryotherapy or heat therapy). Moreover, the gel may be adapted to facilitate activation of the actuator. Namely, if the actuator is of a conducting-polymer type, a gel-based electrolyte may both facilitate actuation of the actuator and accomplish the thermotherapy.

According to still another preferred embodiment of this aspect of the invention, the pressure transition system is adapted to apply a bias pressure profile to the body part. Hence, an initial pressure profile exists also before actuation of the segments' actuators. Thereby, the workload for the actuators is reduced, and energy may be economized.

According to another aspect of the invention, the object is achieved by the initially described therapeutic garment, wherein the garment includes at least one of the proposed devices. Of course, such a garment is advantageous for the same reasons as the above-described device.

Hence, by means of the invention, a cost efficient solution is attained for applying an external pressure to a human body part, which also is highly flexible. The invention may be optimized for various medical purposes, and for instance be used for treatment or prophylaxis of lymphoedema, deep venous thrombosis, venous leg ulcers, venous insufficiency, arterial ulcers, arterial insufficiency, diabetic foot ulcers, cardiovascular diseases, claudication, burns and sports injuries. The proposed solution may also be used in stress therapy, massage therapy enhanced external counter pulsation therapy and blood pressure monitoring.

Further advantages, advantageous features and applications of the present invention will be apparent from the following description and the dependent claims.

### BRIEF DESCRIPTION OF THE DRAWINGS

The present invention is now to be explained more closely by means of preferred embodiments, which are disclosed as examples, and with reference to the attached drawings.
- Figures 1 a-b: show schematic cross-section views of devices according to embodiments of the invention,
- Figure 2: shows a perspective view of a device according to a first embodiment of the invention,
- Figure 3: shows a perspective view of a device according to a second embodiment of the invention,
- Figures 4a-b: show schematic cross-section views of a first embodiment of a proposed pressure transition system,
- Figures 5a-b: show schematic cross-section views of a second embodiment of a proposed pressure transition system,
- Figures 6a-c: show perspective views of further embodiments of the proposed pressure transition system,
- Figure 7: shows a cross-section view of the pressure transition system according to a particular embodiment of the invention, and
- Figures 8-11: illustrate examples of therapeutic garments including the proposed device.

### DESCRIPTION OF PREFERRED EMBODIMENTS OF THE INVENTION

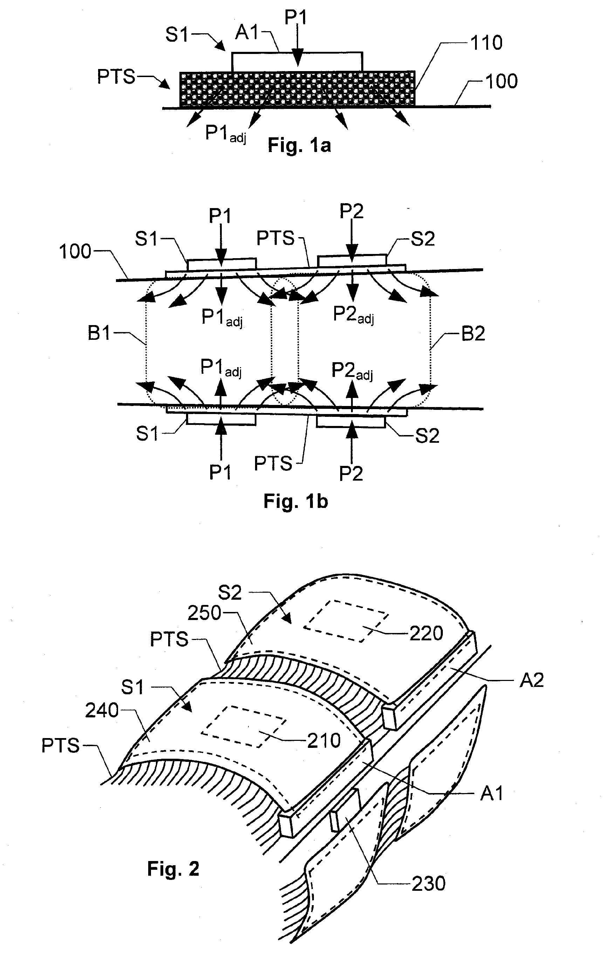

Figure 1 a shows schematic cross-section view of a device according to one embodiment of the invention for exerting an external pressure to a human body part 100. The device includes a segment S1, which is adapted to at least partially enclose the body part 100 in a form-fitting manner. The segment S1 contains a controllable active-material based actuator A1 (e.g. of electroactive ceramics or polymer, conducting-polymer, carbon-nano-tube or electroactive-gel type) that in response to a control signal is adapted to cause the segment S1 to apply a basic pressure profile P1 to the body part 100. A pressure transition system PTS of the device is adapted to redistribute this basic pressure profile P1 into an adjusted pressure profile P1_{ad}, which is different from the basic pressure profile P1. Thus, in response to a control signal in respect of the segment S1, the adjusted pressure profile P1_{adj} is applied to the body part 100.

According to one embodiment of the invention, the pressure transition system PTS is an underlayer that is located between the segment S1 and the body part 100. Further, the pressure transition system PTS may include an auxetic-foam composite or alternative deformable microcellular structure, and have one or more walls of a stretchy fabric, which allow the system PTS to expand in the circumferential and/or axial directions, so that the external basic pressure profile P1 causes a deformation of the system PTS, and as a result an adjusted pressure profile P1_{adj} is exerted on the body part 100.

The pressure transition system PTS may also include drug containing pockets (here generally illustrated by means of white circles). In this case, the system PTS is also adapted to administer a transport of any drug substance in these pockets to the body part 100, for instance in connection with an applied basic pressure profile P1. The drug substance may contain various topical agents for slow release application to the body part 100. Such topical agents may soften or moisturize the tissues in the body part 100 to prevent cracking and maintain or improve the overall health of the patient's skin. Alternatively, the drug substance may contain benzopyrones, flavonoids, coumarin, terpenses etc. for slow release into the underlying body part 100. Moreover, the drug substance may be an antibacterial agent, which helps in preventing infection of a wound site in the body part 100.

According to one embodiment of the invention, the drug substance is a gel, which is adapted to perform a thermotherapy on the body part 100 (e.g. a cryotherapy for pain relief, or a heat therapy to promote tissue healing). If the actuator A1 is based on an active material that requires an electrolyte and if a thermotherapy of the body part 100 is desired, it is preferable to let a gel based electrolyte play dual rolls in both operating the actuator A1 and accomplishing the cryotherapy.

Figure 1b shows a schematic cross-section view of a device according to one embodiment of the invention where the device includes (at least) two segments S1 and S2. A first segment S1 at least partially encloses a first portion B1 of a body part 100 and a second segment S2 at least partially encloses a second portion B2 of the body part 100. The pressure transition system PTS is here adapted to redistribute pressure profiles between the first and second segments S1 and S2. Specifically, this means that if the first segment S 1 receives a control signal which causes the segment S1 to generate a first basic pressure profile P1, the pressure transition system PTS applies a first adjusted pressure profile P1_{adj} to at least a part of the second portion B2 of the body part 100. Correspondingly, if the second segment S2 receives a control signal which causes this segment S2 to generate a second basic pressure profile P2, the pressure transition system PTS applies a second adjusted pressure profile P2_{adj} to at least a part of the first portion B1 of the body part 100. Hence, in response to control signals in respect of the segments S1 and S2 relatively smoothed-out, or fuzzy, pressure profiles are applied to the body part 100. This is advantageous both from a medical and a patient-comfort point-of-view.

According to one embodiment of the invention, the pressure transition system PTS is adapted to apply a bias pressure profile to the body part, such that the body part 100 is exerted to an initial pressure profile also before any of the basic pressure profiles P1 or P2 are applied. The bias pressure profile may be attained passively due to the pressure transition system PTS being stretchy. Then, the segments S1 and S2 may operate "on top of" this bias pressure profile to provide adjustments and/or dynamic therapies. Thereby, the pressure transition system PTS not only redistributes the basic pressure profiles P1 or P2 but also modifies the magnitude of the average pressure. For example, the segments S1 and S2 may apply basic pressure profiles P1 and P2 of 20 mmHg to the pressure transition system PTS, which already applies 20 mmHg to the body part 100. As a result, a pressure in the order of 40 mmHg is applied to the body part 100.

As can be seen in the figure 1b, at each body cross section enclosed by the device, the pressure transition system PTS is positioned between a first surface defined by the first and second segments S1 and S2, and a second surface defined by the body part 100. Additionally, the pressure transition system PTS extends over the first and second portions B1 and B2 of the body part 100.

Figure 2 shows a perspective view of a device according to a first embodiment of the invention. Here, two segments S1 and S2 are shown. However, according to the invention, the device may include any number of segments larger than two. The pressure transition system PTS is at least positioned between the first and second segments S1 and S2. Thereby, the pressure transition system PTS may bridge over pressure profiles and tension forces from one segment to another, i.e. from S1 to S2, from S2 to S1, etc. Here, different degrees of coupling between the segments may be attained depending upon which fiber directions that are chosen for a fabric used in the pressure transition system PTS.

As mentioned above, according to the invention, each segment S1 and S2 includes an actuator A1 and A2 respectively. Here, the segments S1 and S2 include a respective strap member 240 and 250, and the actuators A1 and A2 are located at one end of each segment. The actuators A1 and A2 are further attached to the strap members 240 and 250, which at least partially enclose the body part 100. In response to control signals, the actuators A1 and A2 are adapted to pull the strap members 240 and 250, thus accomplish tension forces relative to the body part 100. According to the invention, many different forms of actuators may produce such tension forces. For example bending, spring, wrinkle, bellows, laminates, friction drives, linear stack piezoceramic (or c-block) and knitted fiber actuators may be used.

Nevertheless, in response to a respective control signal, the actuators A1 and A2 of figure 2 adjust their morphology so that a tangential movement T of segments S1 and/or S2 occurs. As a result, a radial pressure is exerted on the body part 100. For energy efficiency reasons, it is preferable that the actuators A1 and A2 be adapted to maintain their adjusted morphologies also after that the control signals have ceased, i.e. that the control signals merely instigate the morphology change.

According to a first alternative embodiment of the invention, the pressure transition system PTS is exclusively positioned between the segments S1 and S2. This design is preferable if a very slim device profile is important. However, according to a second alternative embodiment of the invention, the pressure transition system PTS also extends underneath the segments S1 and S2. In this case it is further preferable if the pressure transition system PTS has a low-friction surface towards the segments S1 and S2, so that smooth tangential movements of the segments' strap members are enabled.

According to this second alternative embodiment of the invention, the pressure transition system PTS may include sensor elements 210 and 220, which are adapted to register relevant parameters, and transmit data signals reflecting these parameters to a control unit for analysis.

For example, the sensor elements 210 and 220 may be adapted to register pressure, and in this case the elements can take the form of thin film force sensors (e.g. capacitive, piezoresistive, piezoelectric, varying contact or Quantum Tunneling Composite - QTC). If, on the other hand, the sensor elements 210 and 220 are intended to monitor the local circumference of the body part 100, the sensor element 230 may instead take the form of a resistive strip, an interdigitated electrode with contacts, or similar sensor which surrounds the body part 100. The sensor elements 210 and 220 may also be responsible for measuring physiological parameters, such as heart rate, galvanic skin response, electromyogram - EMG, blood oxygen levels, exudates extraction rates.

In the embodiment illustrated in figure 2, the pressure transition system PTS includes a sensor element 230 that is adapted to register a parameter expressing an environmental condition in proximity to the body part 100, e.g. temperature, airflow, humidity or contamination. Namely, these types of environmental conditions may also influence what is an ideal behavior of the proposed device. Thus, based on data signals from the sensor elements 210 and 220 and/or the sensor element 230, a treatment profile executed by the device may be adjusted.

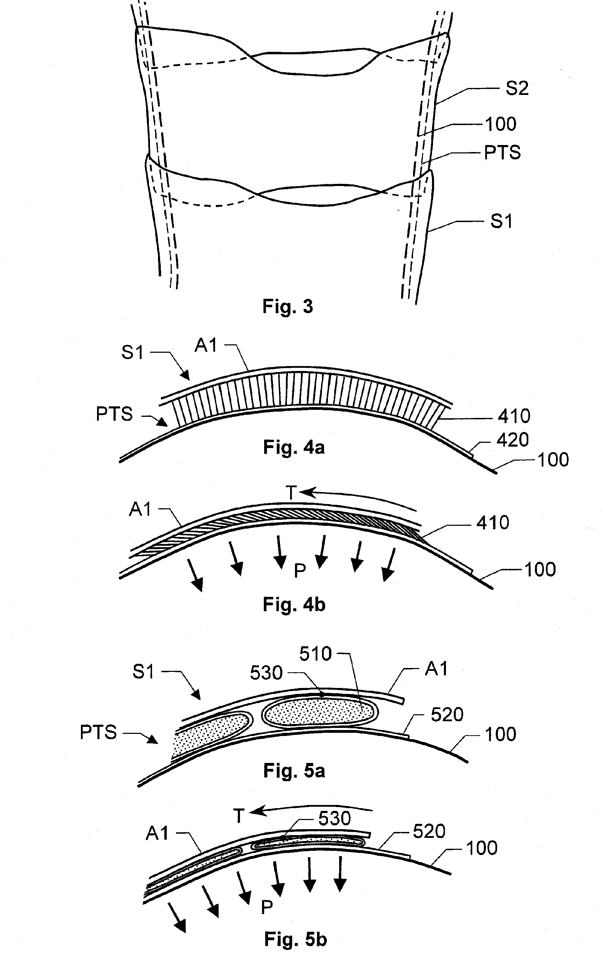

Figure 3 shows a perspective view of a device according to a second embodiment of the invention. Here, each of a number of segments S1, S2, etc. at least partially encloses a body part 100. Moreover, the segments are arranged such that a portion of one segment S1 covers a portion of a neighboring segment S2, and so on. Thereby, analogous to the embodiment described above with reference to figure 1b, relatively smoothed-out, or fuzzy, pressure profiles may be applied to the body part 100 in response to control signals in respect of the segments S1 and S2. Moreover, to redistribute these pressure profiles a pressure transition system PTS is located between the segments and the body part 100. Preferably, the pressure transition system PTS has a low-friction surface towards the segments S1 and S2, so that smooth tangential movements of the segments S1 and S2 are allowed relative to the pressure transition system PTS.

Figures 4a-b show two cross-section views of one embodiment of the proposed pressure transition system PTS.

Here, the pressure transition system PTS includes a number of collapsible ribs 410 which are positioned between at least one segment S1 and a particular portion of the body part 100 when the device is fitted on the body part 100. Preferably, a cover layer 420 separates the ribs 410 from the body part 100. The ribs 410 extend along a general central axis of the body part 100. Hence, in these cross-section views, we only see the section profile of the ribs 410. In response to a control signal, an actuator A1 of the segment S1 is adapted to cause a tangential movement T of the segment S1 relative to the body part 100 (see figure 4b). In response to the movement T, in turn, the ribs 410 are adapted to fold, such that when folded the ribs 410 exert a radial pressure P on the particular portion of the body part 100.

Figures 5a-b show two cross-section views of another embodiment of a proposed pressure transition system PTS. Also in this case, the pressure transition system PTS is adapted to transform a tangential movement T into a resulting radial pressure P on a body part 100. Here, however, the pressure transition system PTS includes at least one flexible chamber 510, which is positioned between at least one segment S1 and the body part 100 when the device is fitted on the body part 100. An actuator A1 of the segment S1 is adapted to cause the tangential movement T of the segment S1 relative to the body part 100 in response to a control signal. The tangential movement T, in turn, deforms the flexible chamber 510, so that the chamber 530 causes a radial pressure P on the body part 100, preferably via an underlayer 520. The chamber 510 has at least one attachment point 530 to the segment S1. When the segment S1 slides over the body part 100 the attachment point 530 follows this movement, and the chamber 510 is compressed. The chamber 510 may contain any flexible medium, such as a gas, a gel or a liquid. In any case, chamber 510 has elastic walls, which according to a preferred embodiment of the invention are made of an anisotropic material. Thereby, the chamber 510 may be arranged relative to the body part 100 when the device is fitted thereto, such that the chamber 510 is relatively stretchable in a circumferential direction of the body part 100 and relatively stiff in a direction along a general central axis of the body part 100. Thus, the radial pressure P may be well distributed over the body part 100. At the same time, the pressure transition system PTS can be soft in the circumferential direction, so that fit and patient comfort is enhanced.

Generally, the tension-force to pressure transduction embodiments illustrated in the figures 4a-b and 5a-b may provide useful designs whenever a slim, robust and energy efficient device is desired.

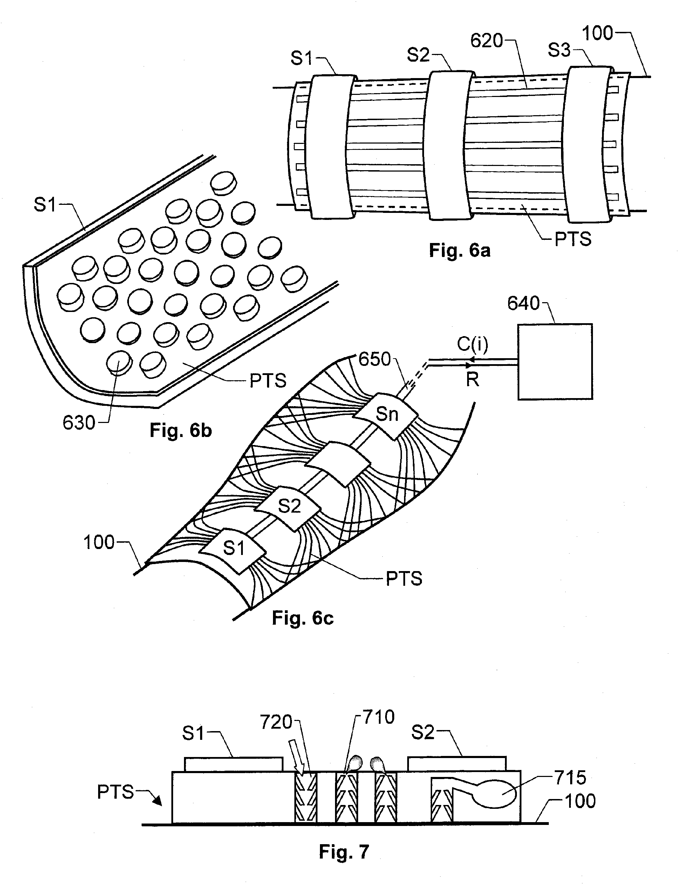

Figure 6a shows a perspective view of another embodiment of the proposed pressure transition system PTS, which includes a number of protrusions in the form of rigid ribs 620. These ribs 620 are adapted to be positioned between at least one segment S1, S2 and S3 respectively and a particular portion of the body part 100. The ribs 620 are adapted to extend along a general central axis of the body part 100 and to convert the basic pressure profile of the segments S1, S2 and S3 into a non-uniform pressure profile to the particular portion of the body part 100. Thus, a peak pressure ridge of the non-uniform pressure profile is produced for each rib, and the pressure ridges are defined by the positioning of the ribs 620 relative to the body part 100. More important, however, by means of the ribs 620 pressure profiles applied by the segments S1, S2 and S3 are distributed across the body part 100. Preferably, the ribs 620 may be sewn into a soft backing material, so that the entire structure can easily expand in the radial direction (e.g. to accommodate a wide range of patient limb sizes) while being stiff in the axial direction (i.e. along the body part 100). For illustrative purposes, the segments S1, S2 and S3 have here been separated more than what is normally preferable.

Figure 6b shows a perspective view of an alternative embodiment of the proposed pressure transition system PTS, where instead the protrusions are cylindrical bulges 630. The bulges 630 are adapted to be positioned between at least one segment S1 and a particular portion of the body part when the device is fitted on the body part. Analogous to the above-mentioned ribs, the bulges 630 are adapted to convert the basic pressure profile of the segment S1 into a non-uniform pressure profile to the particular portion of the body part. Here, however, each bulge 630 causes a circular pressure peak. Such pressure peaks are particularly suitable when treating lymphoedema.

Figure 6c shows yet another perspective view of a device according to one embodiment of the invention. The device includes a number of segments S1, S2, ..., Sn, which are arranged linearly along a body part 100, such as an arm or a leg. Analogous to the embodiment shown in figure 6a. for illustrative purposes the segments S1, S2 and S3 have also here been separated more than what is normally preferable. Nevertheless, each segment S1, S2, ..., Sn is associated with a pressure transition system PTS which encloses the body part 100 and has fiber directions according to the curved lines. Moreover, the pressure transition systems PTS overlap partially, such that some portions of the body part 100 are covered by more than one pressure transition system PTS. For instance, a majority of the body part 100 may be covered by at least two different pressure transition systems PTS. This configuration results in that an activation of a first segment S1 causes a pressure to be applied to portions of the body part 100 which may also be pressurized via a second segment S2, and so on. Hence, smoothed-out, or fuzzy, pressure profiles may be applied to the body part 100 in response to control signals C(i) in respect of the segments S1, S2, ..., Sn.

According to a preferred embodiment of the invention, a control unit 640 produces a respective control signal C(i) to each of segment S1, S2, ..., Sn. Preferably, these control signals C(i) are distributed via a common signal delivery system 650. The control unit 640 is adapted to vary the control signals C(i) over time, so that a treatment profile is implemented with respect to the body part 100. The treatment profile may involve producing repeated cycles of variations between relatively high and relatively low basic pressure profiles by means of each segment S1, S2, ..., Sn.

The treatment profile, in turn, may be adaptive in response to a manipulation signal that either is an external signal, or is produced by the device itself. For example, as mentioned above with reference to the figure 2, one or more sensor elements in the segments S1, S2, ..., Sn may transmit data signals R to the control unit 640. Consequently, the manipulation signal can be based on such data signals R, so that the treatment profile depends on a current state of the body part 100 and/or the current environmental conditions. Moreover, the data signals R may reflect a patient's posture. Therefore, according to the invention, it is rendered possible to adapt the treatment profile to the posture. For example, if the segments S1, S2, ..., Sn are fitted around the patient's leg, they may be completely relaxed when the patient is lying down (e.g. apply a pressure profile in the range 0-10 mmHg), apply a relatively low graduated pressure profile (e.g. in the range 0-40 mmHg) when the patient is standing up and apply a relatively high graduated pressure profile (e.g. in the range 0-60 mmHg) when the patient is sitting.

According to preferred embodiment of the invention, the control signals C(i) are electrical signals, and the segments S1, S2, ..., Sn have actuators whose morphology is electrically adjustable. Moreover, the adjustments of the actuator morphologies are preferably only instigated by the control signals C(i) (i.e. no control signal is necessary to maintain an adjusted morphology).

Naturally, according to the invention, the control unit 640 may be connected to any of the proposed segments and pressure transition systems, i.e. not only the elements of embodiment shown in figure 6c.

Figure 7 shows a cross-section view of the pressure transition system PTS according to one embodiment of the invention, where the pressure transition system PTS includes a number of moisture passages 710 schematically illustrated as tubes with internal flanges. Each moisture passage 710 is adapted to receive exudates from the body part 100, and thus assist in keeping the skin relatively dry.

According to one preferred embodiment of the invention, the pressure transition system PTS includes one or more liquid receptacles 715, and the moisture passages 710 are adapted to transport any received exudates from the body part 100 this/these receptacle/s 715 concomitantly with repeating cycles of a treatment profile executed by means of segments S1 and S2 associated with the pressure transition system PTS. Preferably, the moisture passages 710 and liquid receptacles 715 are accomplished by means of air pockets of an open-celled foam. Thus, these elements' physical configuration is quite dissimilar from what is illustrated in figure 7, however their function is identical thereto.

According to another preferred embodiment of the invention, the pressure transition system PTS includes a number of air channels 720 which are adapted to allow air to pass to the body part 100. Analogous with the moisture passages 710 and the liquid receptacles 715, the air channels 720 may also be adapted to operate concomitantly with the repeating cycles of the treatment profile executed by means of the segments S1 and S2, so that air is exchanged more efficiently between the body part 100 and a local environment outside thereof. Moreover, open-celled foam openings may also constitute the air channels 720.

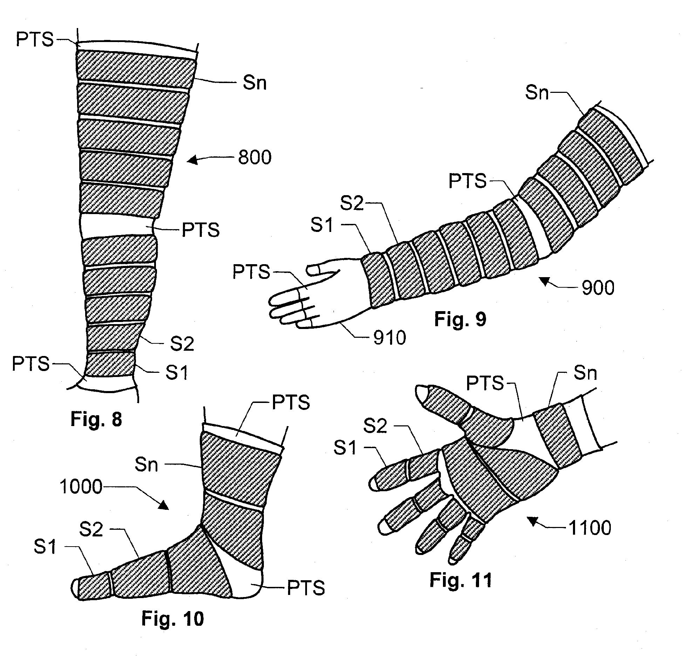

Figures 8-11 1 illustrate examples of therapeutic garments 800, 900, 1000 and 1100 including the proposed device.

Figure 8 shows a leg garment 800, where a plurality of segments S1, S2, ..., Sn are adapted to enclose both the upper and the lower part of a patients leg. An extensive pressure transition system PTS is adapted to redistribute basic pressure profiles generated by the segments S1, S2, ..., Sn, so that the entire leg is exerted to adjusted pressure profiles, for example also at joint portions of the leg that are not covered by any segments.

Figure 9 shows an arm garment 900, where a plurality of segments S1, S2, ..., Sn are adapted to enclose a patient's forearm and over arm. Also here an extensive pressure transition system PTS is adapted to redistribute basic pressure profiles generated by the segments S1, S2, ..., Sn. Thereby, for instance, an elbow portion which for flexibility reasons is not covered by segments may be exerted to pressure. Additionally, the pressure transition system PTS is adapted to extend over the patient's hand in the form of a compression glove, so as to prevent lymphatic fluids from pooling in the hand.

Figures 10 and 11 show garments 1000 and 1100 for exerting external pressures to a patient's foot and hand respectively. In both these cases a plurality of segments S1, S2, ..., Sn are adapted to enclose a said extremities, and a pressure transition system PTS is adapted to redistribute basic pressure profiles generated by the segments S1, S2, ..., Sn. Thus, basic pressure profiles may be smoothed out and also portions which are not covered by segments may be pressurized.

The term "comprises/comprising" when used in this specification is taken to specify the presence of stated features, integers, steps or components. However, the term does not preclude the presence or addition of one or more additional features, integers, steps or components or groups thereof.

The invention is not restricted to the described embodiments in the figures, but may be varied freely within the scope of the claims.

## Claims

1. A device for exerting an external pressure to a human body part (100), comprising a number of segments (S1, S2, ..., Sn) adapted to at least partially enclose the body part (100) in a form-fitting manner, each segment containing a controllable active-material based actuator (A1, A2) adapted to cause the segment to apply a basic pressure profile (P1) to the body part (100) in response to a control signal (C(i)),
**characterized in that**
the device further comprises a pressure transition system (PTS) adapted to redistribute the basic pressure profile (P1) of at least one of said segments (S1), such that in response to a control signal (C(i)) in respect of said at least one segment (S1) an adjusted pressure profile (P1_{adj}) different from the basic pressure profile (P1) is applied to the body part.

2. A device according to claim 1, **characterized in that** the device comprises at least two segments (S1, S2, ..., Sn) of which a first segment (S1) is adapted to at least partially enclose a first portion (B1) of the body part (100) and a second segment (S2) is adapted to at least partially enclose a second portion (B2) of the body part (100), the pressure transition system (PTS) is adapted to redistribute pressure profiles (P1, P2) between the first and second segments (S1, S2), such that
a control signal (C(i)) in respect of the first segment (S1) causes the pressure transition system (PTS) to apply an adjusted pressure profile (P1_{adj}) to at least a part of the second portion (B2) of the body part (100), and
a control signal (C(i)) in respect of the second segment (S2) causes the pressure transition system (PTS) to apply an adjusted pressure profile (P2_{adj}) to at least a part of the first portion (B1) of the body part (100).

3. A device according to claim 2, **characterized in that** the pressure transition system (PTS) is adapted to be positioned between the first and second segments (S1, S2) when the device is fitted on the body part (100).

4. A device according to any one of the claims 2 or 3, **characterized in that** the pressure transition system (PTS) is adapted to be positioned between a first surface defined by the first and second segments (S1, S2) and a second surface defined by the body part (100), and the pressure transition system (PTS) extends over the first and second portions (B1, B2) of the body part (100) when the device is fitted on the body part (100).

5. A device according to claim 4, **characterized in that** the pressure transition system (PTS) has a low-friction surface towards the first and second segments (S1, S2), and said surface is adapted to allow a smooth tangential movement of the first and second segments (S1, S2) relative to the pressure transition system (PTS).

6. A device according to any one of the preceding claims, **characterized in that** the device comprises at least two segments (S1, S2, ..., Sn) of which a first segment (S1) is adapted to at least partially enclose a first portion (B1) of the body part (100) and a second segment (S2) is adapted to at least partially enclose a second portion (B2) of the body part (100), and the first and second segments (S1, S2) are arranged such that a portion of the first segment (S1) covers a portion of the second segment (S2) when the device is fitted on the body part (100).

7. A device according to any one of the preceding claims, **characterized in that** the pressure transition system (PTS) comprises a number of collapsible ribs (410) adapted to extend along a general central axis of the body part (100) and be positioned between at least one of said segments (S1) and a particular portion of the body part (100) when the device is fitted on the body part (100), an actuator (A1) of each of said at least one segment (S1) is adapted to cause a tangential movement (T) of said at least one segment (S1) relative to the body part (100), and the collapsible ribs (410) are adapted to fold in response to said movement (T) such that when folded the ribs (410) exert a radial pressure (P) on the particular portion of the body part (100).

8. A device according to any one of the preceding claims, **characterized in that** the pressure transition system (PTS) comprises at least one flexible chamber (510) adapted to be positioned between at least one of said segments (S1) and a particular portion of the body part (100) when the device is fitted on the body part (100), an actuator (A1) of each of said at least one segment (S1) is adapted to cause a tangential movement (T) of said at least one segment (S1) relative to the body part (100), and the at least one flexible chamber (510) is adapted to transform said movement (T) into a resulting radial pressure (P) on the particular portion of the body part (100).

9. A device according to claim 8, **characterized in that** the at least one flexible chamber (510) has an elastic wall of an anisotropic material, the at least one chamber (510) is adapted to be arranged relative to the body part (100) when the device is fitted on the body part (100) such that the at least one chamber (510) is relatively stretchable in a circumferential direction of the body part (100) and relatively stiff in a direction along a general central axis of the body part (100).

10. A device according to any one of the preceding claims, **characterized in that** the pressure transition system (PTS) comprises a number of protrusions (620, 630) adapted to be positioned between at least one of said segments (S1) and a particular portion of the body part when the device is fitted on the body part, said protrusions are adapted to convert the basic pressure profile of said at least one segment (S1) into a non-uniform pressure profile to the particular portion of the body part (100).

11. A device according to claim 10, **characterized in that** said protrusions comprise at least one rigid rib (620) adapted to extend along a general central axis of the body part (100) and be positioned between at least one of said segments and a particular portion of the body part when the device is fitted on the body part, and a respective peak pressure ridge of the non-uniform pressure profile is defined by a positioning of each of the at least one rib (620) relative to the body part (100).

12. A device according to any one of the preceding claims,
**characterized in that**
the control signal (C(i)) is an electrical signal,
each of said actuators (A1, A2) has a morphology which is electrically adjustable, and
a change of said morphology is instigated by the control signal (C(i)).

13. A device according to claim 12, **characterized in that** the device comprises a control unit (640) adapted to produce a respective control signal (C(i)) to each of said segments (S1, S2, ..., Sn), and the control unit (640) is adapted to vary the control signal (C(i)) over time to implement a treatment profile with respect to the body part (100).

14. A device according to claim 13, **characterized in that** the treatment profile involves producing repeated cycles of variations between relatively high and relatively low basic pressure profiles by means of each of said segments.

15. A device according to any one of the preceding claims, **characterized in that** the pressure transition system (PTS) comprises a number of moisture passages (710) adapted to receive exudates from the body part (100).

16. A device according to claim 15 when dependent on claim 14, **characterized in that** said moisture passages (710) are adapted to transport any received exudates from the body part (100) to at least one liquid receptacle (715) concomitantly with said repeating cycles.

17. A device according to any one of the preceding claims, **characterized in that** the pressure transition system (PTS) comprises a number of air channels (720) adapted to allow air to pass to the body part (100).

18. A device according to claim 17 when dependent on claim 14, **characterized in that** the air channels (720) are adapted to exchange air between the body part (100) and a local environment outside the device concomitantly with said repeating cycles.

19. A device according to any one of the claims 13 - 18, **characterized in that** the pressure transition system (PTS) comprises at least one sensor element (210, 220) adapted to register a physiological parameter of the body part (100), and transmit a data signal (R) reflecting this parameter to the control unit (640).

20. A device according to any one of the claims 13 - 19, **characterized in that** the pressure transition system (PTS) comprises at least one sensor element (230) adapted to register a parameter expressing an environmental condition in proximity to the body part (100), and transmit a data signal (R) reflecting this parameter to the control unit (640).

21. A device according to any one of the claims 19 or 20, **characterized in that** the treatment profile is adaptive in response to at least one manipulation signal.

22. A device according to claim 21, **characterized in that** at least one of the at least one manipulation signal is based on at least one of said data signals (R).

23. A device according to any one of the preceding claims, **characterized in that** the pressure transition system (PTS) comprises at least one pocket adapted to contain a drug substance and administer a transport of this substance to the body part (100).

24. A device according to claim 23, **characterized in that** the drug substance is an antibacterial agent.

25. A device according to claim 23, **characterized in that** the drug substance is a gel adapted to perform a thermotherapy on the body part (100).

26. A device according to claim 25, **characterized in that** the gel is adapted to facilitate activation of said actuator (A1).

27. A device according to any one of the preceding claims, **characterized in that** the pressure transition system (PTS) is adapted to apply a bias pressure profile to the body part (100).

28. A therapeutic garment (800, 900, 1000, 1100) adapted to cover at least one appendage of a human body, **characterized in that** the garment comprises at least one device according to any one of the claims 1 - 27.
